(19) Europäisches Patentamt
European Patent Office
Office européen des brevets

(11) Publication number: 0 208 662 B1

(12) EUROPEAN PATENT SPECIFICATION

(45) Date of publication of patent specification: 14.12.88

(51) Int. Cl.[4]: C 07 C 101/30, C 07 C 99/00, C 07 D 303/40, C 12 P 7/62

(21) Application number: 86830162.3

(22) Date of filing: 11.06.86

(54) **Process for manufacturing r(-)-norcarnitine tert-butyl ester.**

(30) Priority: 28.06.85 IT 4829785

(43) Date of publication of application: 14.01.87 Bulletin 87/03

(45) Publication of the grant of the patent: 14.12.88 Bulletin 88/50

(84) Designated Contracting States: AT BE CH DE FR GB LI LU NL SE

(56) References cited:
EP-A-0 169 176
FR-A-2 184 090

(73) Proprietor: Sigma-Tau Industrie Farmaceutiche Riunite S.p.A.
47, Viale Shakespeare
I-00144 Rome (IT)

(72) Inventor: Tinti, Maria Ornella
81, Via Ernesto Basile
I-00128 Rome (IT)

(74) Representative: Fassi, Aldo, Dr.
c/o Sigma-Tau Industrie Farmaceutiche Riunite S.p.A. Via Pontina, Km. 30, 400
I-00040 Pomezia (Roma) (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 208 662 B1

Courier Press, Leamington Spa, England.

## Description

The present invention relates to a process for preparing R(−)-norcarnitine tert-butyl ester (Tert-butyl 4-dimethylamino-3(R)-hydroxy butyrate) having formula (I)

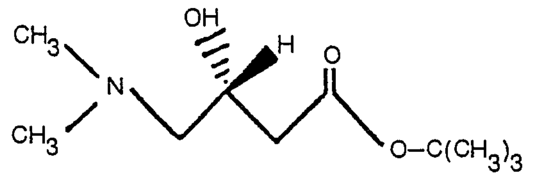

(I)

(I) is a useful intermediate in the preparation of R(−)-carnitine and R(−)-carnitine derivatives, such as alkanoyl R(−)-carnitines, whose various therapeutical utilizations are known.

Tert-butyl ester of norcarnitine contains an asymmetric carbon atom. This molecular species thus exists in two optically active stereoisomer forms, the R stereoisomer (its configuration is illustrated by formula (I)) and the S stereoisomer (its configuration is illustrated for formula (I')), and in the optically inactive R,S-racemic mixture.

Recently, particular emphasis has been placed on the importance of utilizing for therapeutical applications R(−)-carnitine and R(−)-carnitine derivatives exclusively, carefully avoiding the presence of the S stereoisomer. This latter exerts, in fact, an inhibiting action on the R stereoisomer (which is on the other hand the "naturally" occurring carnitine found in muscular tissues and myocardium of mammals). In addition to both chemical and microbiological methods for resolving racemic mixtures which aim at isolating the desired R stereoisomer from the S stereoisomer, chemical and microbiological methods have been developed for selectively synthesizing a carnitine precursor which already presents the desired R configuration, typical of the natural carnitine.

In the Italian patent application 48504A84 filed on July 4, 1984 in the name of the same applicant of the present application (see also the corresponding EP application 0169176), tert-butyl ester of R,S-norcarnitine and a process for its preparation have been disclosed and claimed. This product (or, optionally, a derivative thereof) must, therefore, be subjected to the above-mentioned resolution procedures in order to isolate the desired R isomer from the S isomer.

The process of this invention allows R(−)-norcarnitine tert-butyl ester to be obtained directly and selectively, without attendant formation of the S isomer.

Therefore, compound (I) shall be directly utilized for producing R(−)-carnitine or any derivative thereof.

The process of the present invention is illustrated by the following reaction scheme.

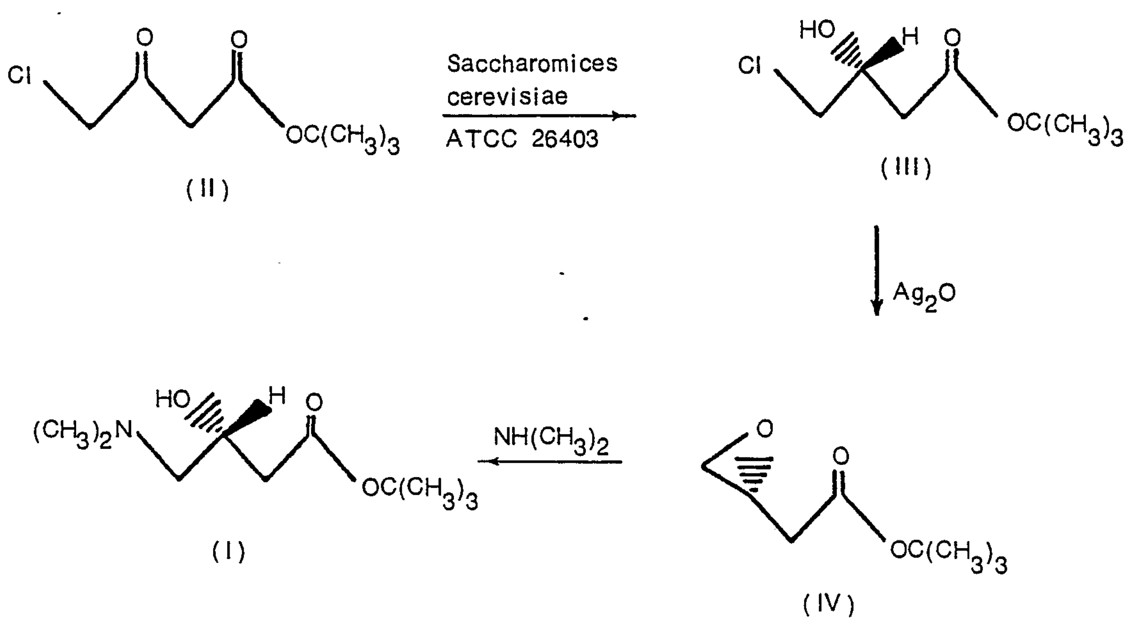


More specifically, the process of the present invention comprises the steps of:

(1) converting tert-butyl-4-chloro-acetoacetate (II) into tert-butyl-4-chloro-3(R)-hydroxy butyrate (III) in the presence of Saccharomices cerevisiae ATCC 26403;

(2) converting (III) into the corresponding epoxide (IV), by keeping a reaction mixture comprising a solution of (III) in an anhydrous organic solvent and silver oxide, at molar ratio (III): $Ag_2O$ 1:3, in an atmosphere of a dry inert gas, at the reflux temperature of the mixture, for 40—48 hours;

(3) reacting a solution of the epoxide (IV) in a lower alkanol with a solution of dimethylamine in a lower alkanol at molar ratio (IV); dimethylamine comprised between 1:09 and 1:1.1, keeping the reaction mixture at about 5—30°C for about 3—8 hours in an atmosphere of inert gas, thus obtaining (I); and (4) isolating (I) from the reaction mixture of step (3) by concentrating the mixture under vacuum and taking up the residue with a water insoluble solvent, extracting with a diluted acid solution and then alkalinizing to pH about 12, dehydrating the resulting organic phase and removing the organic solvent via distillation under reduced pressure.

Preferably, the organic solvent of step (2) is selected from dimethoxyethane and ethyl ether, the lower alkanol of step (3) is methanol and the water-insoluble organic solvent of step (4) is selected from ethyl ether and ethyl acetate.

Example

(1) Preparation of tert-butyl-4-chloro-3(R)-hydroxy butyrate (III) — Surface growth from a week old agar slant of Saccharomices cerevisiae ATCC 26403, grown on the yeast extract-peptone-dextrose-fatty acid agar medium (E. Schweizer e H. Bolling, Proc. Nat. Acad. Sci. USA (1970) *67,* 660) was suspended in a 0.85% saline solution. One ml portions of this suspension were used to inoculate a 250 ml Erlenmeyer flask (F-1 stage) containing 50 ml of the following medium (YEPD-FA):

| | |
|---|---|
| Sucrose | 20 g |
| Peptone | 20 g |
| Yeast extract | 10 g |
| $K_2HPO_4$ | 5 g |
| $KH_2PO_4$ | 5 g |
| Myristic acid | 0.07 g |
| Palmitic acid | 0.07 g |
| Stearic acid | 0.07 g |
| Distillled water, q.s | 1 litre |
| (Sterilized for 15 min at 30 p.s.i., 2,1 $Kg/cm^2$) | |

The flask was incubated at 25°C on a rotary shaker (250 cycles/min—5 cm radius) for 24 hours, after which this flask served as the inoculum for a 2000 ml Erlenmeyer flask (F—2 stage) containing 500 ml of the same liquid medium (YEPD—FA). After 20 hours of incubation on a rotary shaker, 1.2 grams of tert-butyl-4-chloroacetoacetate (diluted to 3 times its original volume with anhydrous EtOH) was slowly added using a Harvard infusion pump (Model 940) at a rate of 3 ml over a 24 hour period. After 24 hours, sucrose was then simultaneously infused into the fermentaion medium at a rate of 7.1 ml (30% sucrose solution) over 24 hours. The continuous slow addition of substrate was terminated after a total of 4.8 g of tert-butyl-4-chloroacetoacetate was added (fourth day). However, sucrose was continuously infused into the flask until the fifth day.

After 5 days, the content (F—2) was exhaustively extracted with three 400 ml portions of ethyl acetate. The ethyl acetate was dried over $Na_2SO_4$ and evaporated yielding 3.6 g of an oily residue. The residue was chromatographed over a silica gel (MN-Kieselgel 60) column. The column was eluted with Skelly B-ethyl acetate 8:1) to yeild 1.9 g of tert-butyl-4-chloro-3(R)-hydroxybutyrate,

$$[\alpha]_D^{23} = +18.09°, (c = 5.1, CHCl_3).$$

(2) Preparation of tert-butyl R—3,4-epoxybutyrate

To a solution of 7.8 g (0.04 moles) of tert-butyl-4-chloro-—3(R)-hydroxybutyrate in 80 ml of anhydrous dimethoxyethane, 18.4 g (0.8 moles) of silver oxide were added under a nitrogen atmosphere. The mixture was kept under stirring overnight at 85°C. To the mixture, further 10 g (0.04 moles) of silver oxide were added and the resulting mixture was refluxed under stirring overnight.

The resulting solution was filtered and the solvent distilled off under reduced pressure (about 50 mm Hg) yielding 5 grams of a yellow liquid corresponding to the title compound.

$$[\alpha]_D^{25} = +23° (c = 1, CHCl_3).$$

NMR $CDCl_3$ δ3.2 (m, 1H, —CH—); 2.8 (t 1H, 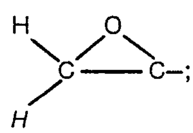

2.4 (m, 3H, 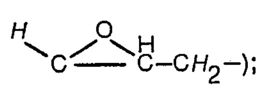 ; 1.4(s, 9H, —$(CH_3)_3$).

TLC silica gel
Eluent petroleum ether: ethyl acetate 5:1
Rf 0.6

| | |
|---|---|
| Gaschromatography | |
| Column | SE 30 (3%) on 80/100 Chromosorb w AW—DMCS (length 1.5 m; inner diameter 4 mm; glass |
| Column temperature | isotherm at 100°C |
| Injector temperature | 140°C |
| Detector temperature | 160°C (FID) |
| Gas rate | $N_2$50 ml/min |
| Chart speed | 0.5 cm/min |
| Retention time | tert-butyl-3,4 epoxybutyrate ≏ 2 min, tert-butyl 4-chloro, 3(R)-hydroxybutyrate ≏ 4 min |

(3) Preparation of R(−)-norcarnitine tert-butyl ester

A solution of epoxide (1g; 6.3 mmoles) in 3 ml of anhydrous methanol was kept under a nitrogen blanket and cooled to 5—10°C. 680 mg of 39% $Me_2NH$ in methanol (5.9 mmoles) were added and the temperature was allowed to rise to 25°C, while stirring was continued for 3 hours. The solvent was then evaporated under reduced pressure and an oily product was obtained which was dissolved in 6 ml of ether and subsequently extracted 3 times with 2 ml of cold 5% HCl. Extract's pH was adjusted to 12 with NaOH and the basic solution was extracted 3 times with 3 ml of ethyl acetate, which was then washed with 2 ml $H_20$. The organic phase was dehydrated over $Na_2SO_4$ and the solvent distilled off under reduced pressure.

700 mg of R(−)-norcarnitine tert-butyl ester were obtained.

Yield: 55%.

The Compound showed Rf 0.7 on silica gel plates with $CHCl_3MeOH/NH_3$ 80/20/2 and developed with $I_2$; it distilled at 100°C at 0.5 mm Hg as a colourless liquid which rapidly turned to yellow at room temperature.

**Claims**

1. A process for preparing tert-butyl 4-dimethylamino-3(R)-hydroxy butyrate having formula:

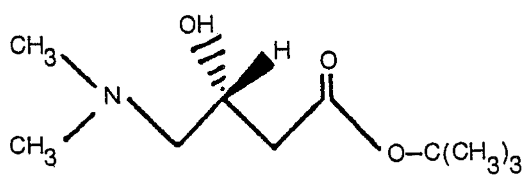

(I)

according to the following reaction scheme:

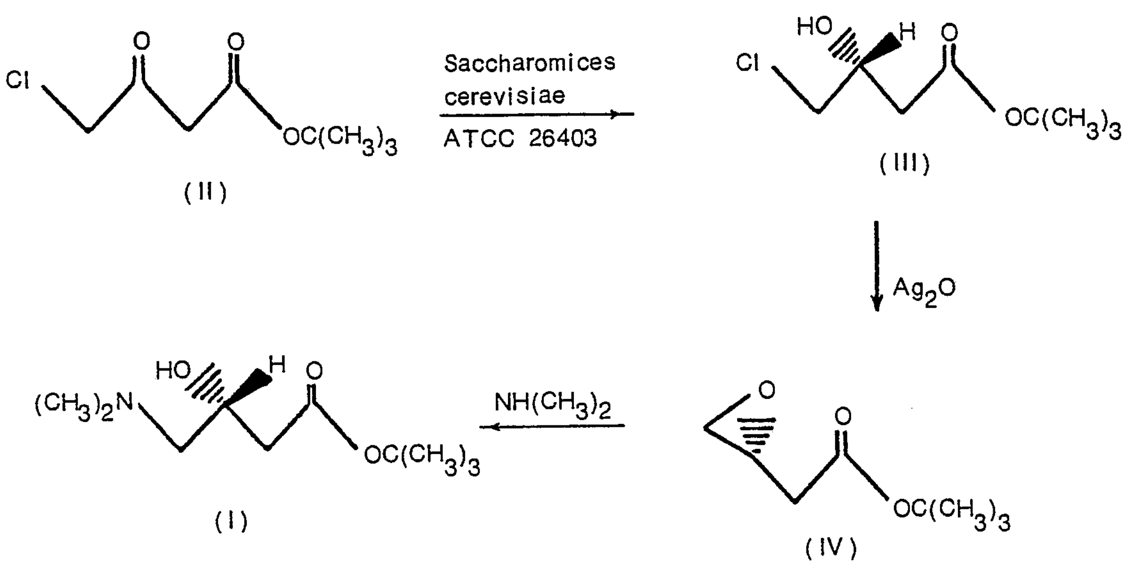


(II) (III) (IV) (I)

which comprises the steps of:

(1) converting tert-butyl-4-chloro-acetoacetate (II) into tert-butyl-4-chloro-3(R)-hydroxy butyrate (III) in the presence of saccharmices cerevisiae ATCC 26403;

(2) converting (III) into the corresponding epoxide (IV), by keeping a reaction mixture comprising a solution of (III) in an anhydrous organic solvent and silver oxide, at molar ratio (III): $Ag_20$ 1:3, in an atmosphere of a dry inert gas, at the reflux temperature of the mixture, for 40—48 hours;

(3) reacting a solution of the epoxide (IV) in a lower alkanol with a solution of dimethylamine in a lower alkanol, at molar ratio (IV): dimethylamine comprised between 1:09 and 1:1.1, keeping the reaction mixture at about 5—30°C for about 3—8 hours in an atmosphere of inert gas, thus obtaining (I); and
(4) isolating (I) from the reaction mixture of step (3) by concentrating the mixture under vacuum and taking up the residue with a water insoluble solvent, extracting with a diluted acid solution and then alkalinizing to pH about 12, dehydrating the resulting organic phase and removing the organic solvent via distilliation under reduced pressure.

2. The process of claim 1, wherein the organic solvent of step (2) is selected from dimethoxyethane and ethyl ether, the lower alkanol of step (3) is methanol and the water-insoluble organic solvent of step (4) is selected from ethyl ether and ethyl acetate.

**Patentansprüche**

1. Verfahren zur Herstellung von tert-Butyl-4-dimethylamino-3(R)-hydroxy-butyrat der Formel

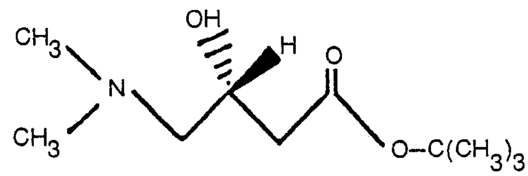

(I)

gemäß dem folgenden Reaktionsschema:

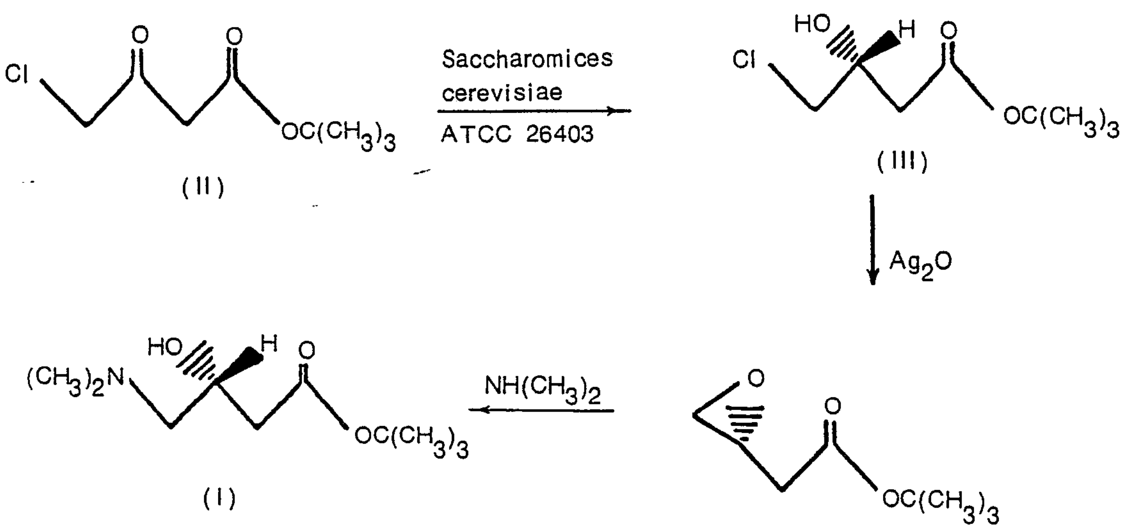


(II) (III) (IV) (I)

welches die folgenden Stufen umfaßt:
(1) Überführung von tert-Butyl-4-chlor-acetoacetat (II) in tert-Butyl-4-chlor-3(R)-hydroxybutyrat (III) in Gegenwart von Saccharomices cerevisiae ATCC 26403;
(2) Überführung von (III) in das entsprechende Epoxid (IV), indem eine Reaktionsmischung aus einer Lösung von (III) in einem wasserfreien organischen Lösungsmittel und Silberoxid in einem Molverhältnis (III): $Ag_2O$ von 1:3, in einer Atmosphäre aus einem trockenen inerten Gas bei der Rückflußtemperatur der Mischung während 40—48 Stunden gehalten wird;
(3) Umsetzung einer Lösung des Epoxids (IV) in einem niederen Alkanol mit einer Lösung von Dimethylamin in einem niederen Alkanol, bei einem Moverhältnis (IV): Dimethylamin in einem Bereich von 1:0,9 bis 1:1,1 wobei die Reaktionsmischung bei etwa 5 bis 30°C während etwa 3 bis 8 Stunden in einer Atmosphäre eines interten Gases gehalten wird, wodurch (I) erhalten wird: und
(4) Isolieren von (I) aus der Reaktionsmischung der Stufe (3) durch Einengen der Mischung unter Vakuum und Aufnehmen des Rückstandes in einem wasserunlöslichen Lösungsmittel, Extrahieren mit einer verdünnten Säurelösung und anschließende Alkalisierung auf etwa pH 12, Dehydratisieren der entstandenen organischen Phase und Entfernen des organischen Lösungsmittels durch Destillation unter vermindertem Druck.

2. Verfahren nach Anspruch 1, bei welchem das organische Lösungsmittel der Stufe (2) aus Dimethoxyethan und Ethylether ausgewählt ist der niedere Alkanol der Stufe (3) Methanol ist und das wasserunlösliche organische Lösungsmittel der Stufe (4) aus Ethylether und Ethylacetat ausgewählt ist.

## Revendications

1. Procédé de préparation de diméthylamino-4 hydroxy-3(R) butyrate de tert-butyle de formule (I)

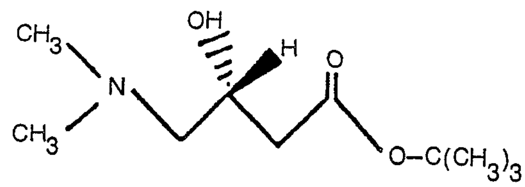

(I)

selon le schéma réactionnel suivant:

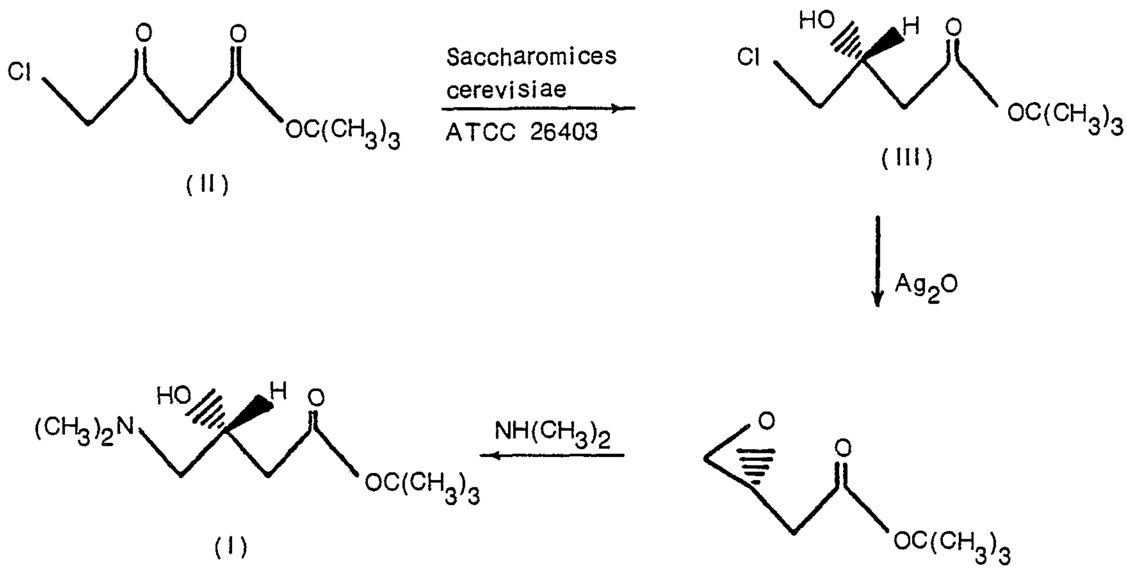


(II) (III) (I) (IV)

qui comprend les étapes consistant à:
(1) transformer le chloro-4 acétylacétate de tert-butyl (II) en chloro-4 hydroxy-3(R) butyrate de tert-butyle (III) en présence de Saccharomices cerevisiae ATCC 26403;
(2) transformer (III) en l'époxyde correspondant (IV), en maintenant pendant 40—48 heures un mélange réactionnel formé d'une solution de (III) dans un solvant organique anhydre et de l'oxyde d'argent selon un rapport molaire (III): $Ag_20$ = 1:3, dans une atmosphère de gaz inerte sec et à la température de reflux du mélange;
(3) faire réagir une solution de l'époxyde (4) dans un alcanol inférieur avec une solution de diméthylamine dans un alcanol inférieur, selon un rapport molaire (IV): diméthylamine compris entre 1:0.9 et 1:1.1, garder le mélange réactionnel à une température d'environ 5—30°C pendant environ 3—8 heures en atmosphère inerte, pour finalement obtenir (I); et
(4) isoler (I) du mélange réactionnel de l'étape (3) par concentration du mélange sous vide, reprise du résidu par un solvant insoluble dans l'eau, extraction avec une solution acide diluée puis alcalinisation à un pH d'environ 12, déshydratation de la phase organique résultante et enfin élimination du solvant organique par distillation sous pression réduite.

2. Procédé selon la revendication 1, dans lequel le solvant organique de l'étape (2) est choisi parmi diméthoxyéthane et éther éthylique, l'alcanol inférieur de l'étape (3) est le méthanol et le solvant organique insoluble dans l'eau de l'étape (4) est choisi parmi éther éthylique et acétate d'éthyle.